Europäisches Patentamt

European Patent Office

Office européen des brevets

(11) Publication number: 0 388 053
A2

# EUROPEAN PATENT APPLICATION

(21) Application number: 90302261.4

(51) Int. Cl.⁵: **C12Q 1/68, G01N 27/26**

(22) Date of filing: 02.03.90

(30) Priority: 07.03.89 US 320230
16.05.89 US 352781

(43) Date of publication of application:
**19.09.90 Bulletin 90/38**

(84) Designated Contracting States:
**AT BE CH DE DK ES FR GB GR IT LI LU NL SE**

(71) Applicant: CHEMBIOMED, LTD.
**2011-94th Street
Edmonton Alberta T6H 4N9(CA)**

(72) Inventor: **Potter, Kathleen**

10634-25A Avenue
**Edmonton, Alberta T6J 4K3(CA)**
Inventor: **Coffin, John**
**922-110A Street
Edmonton, Alberta T6J 6N1(CA)**
Inventor: **Lamontagne, Louis R.**
**3132 44B Avenue
Edmonton, Alberta T6T 1G4(CA)**

(74) Representative: **Goldin, Douglas Michael et al
J.A. KEMP & CO. 14, South Square Gray's Inn
London WC1R 5EU(GB)**

(54) **Speciation of mycobacteria by pulsed-field gel electrophoresis of chromosomal DNA fragments.**

(57) Various strains and species of mycobacteria can be characterized using an improved technique involving digestion of the bacterial chromosome to obtain relatively large DNA fragments and separating the resulting fragments using pulsed-field gel electrophoresis techniques. The resulting patterns are characteristic of particular mycobacteria. The chromosomal DNA is conveniently obtained in situ in a gel plug by embedding cells into the plug and extracting non-DNA cellular components. The plug can then be used directly in the size separation.

FIG. I

# SPECIATION OF MYCOBACTERIA BY PULSED-FIELD GEL ELECTROPHORESIS OF CHROMOSOMAL DNA FRAGMENTS

## Technical Field

The invention relates to characterization of procaryotic organisms by the restriction enzyme fragmentation of their chromosomal DNA. More specifically, the invention concerns speciating mycobacteria using an improved technique for DNA characterization.

## Background Art

The ability to determine the species and strain of mycobacteria is important in clinical analysis of a number of suspected diseases. Mycobacteria are capable of causing a large number of diseases in humans and other animals. For example, Johne's disease, which is associated with chronic fatal diarrhea, is a commonly encountered disease of ruminants, mostly cattle, sheep and goats, and is caused by M. paratuberculosis. M. paratuberculosis has also been found to infect stump-tail Macaque monkeys and has been isolated from the intestines of humans diagnosed with Crohn's disease. Other species involved in infection in humans and animals include M. avium, M. intracellulare and M. scrofulaceum, which are members of the "MAIS" serocomplex.

Clinical and veterinary analysis of suspected mycobacterial isolates has classically involved analysis of phenotypic characteristics, such as animal pathogenicity, growth rate, pigment production, colony morphology, and the homogeneity of a suspension of organisms in media. More precise characterization has been made using characterizing reactions such as nitrate reduction, hydrolysis of Tween 80, arylsulfatase production, catalase production and inactivation, sodium chloride tolerance, urease production, tellurite reduction, niacin production and susceptibility to thiophene-2-carboxylic acid hydrazide. In addition, chromatographic procedures, including gas liquid chromatography and thin layer chromatography have been used to characterize mycobacterial strains based on the fatty acid elution profiles obtained from bacterial lipid extracts.

None of the foregoing methods has proved to be satisfactory due to a number of factors, including the long incubation periods required for growth and the possible instability of the characteristics of isolates upon storage and repeated passages. For example, although mycobactin dependence has been used as a taxonomic characteristic of M. paratuberculosis, upon serial subculture, some isolates lose their mycobactin dependence; also some strains of M. avium are mycobactin-dependent. Serological methods used to characterize members of the MAIS complex have been hampered by a lack of reactivity of many MAIS strains or due to autoagglutination. Based on phenotypic results, it has been uncertain whether M. paratuberculosis is, in fact, a member of the MAIS complex.

Attempts have been made to apply various types of genetic analysis to mycobacteria. (It is known that the genome of mycobacteria ranges from 2.5-4.5 x 10⁹ daltons.) These approaches have included characterization on the basis of G-C content (Wayne, L.G., et al. J Bacteriol (1968) 96:1915-1919); DNA:DNA hybridization in solution (Yoshimura, H.H., et al., J Clin Microbiol (1987) 25:45-51; Bradley, S.G., et al., Am Rev Resp Dis (1972) 106:122-124) and a number of other references which have extensively studied DNA: DNA hybridization patterns using this technique. More closely related to the present invention are approaches which utilize Southern blot probed with mycobacterial DNA (McClure, H.M., et al., J Infectious Diseases (1987) 155:1011-1019; McFadden, J.J:, et al., J Clin Microbiol (1987) 25:796-801; Drake, T.A., et al., J Clin Microbiol (1987) 25:1442-1445; Kiehn, T.E., et al., J Clin Microbiol (1987) 25:1551-1552; Mcfadden, J.J., et al., Mol Microbiol (1987) 1:283-291; Ellner, P.D., et al., J Clin Microbiol (1988) 26:1349-1352; Picken, R.N., et al., Mol and Cell Probes (1988) 2:111-124). Restriction endonuclease patterns of DNA from mycobacteria wherein the DNA has been isolated as a suspension in liquid have also been studied (Collins, D.M., et al., Gen Microbiol (1984) 130:1019-1021; Collins, D.M., et al., Am J Vet Res (1986) 10:2226-2228; Garcia, M.J., et al., J Gen Microbiol (1986) 132:2265-2269; Patel, R., et al., J Gen Microbiol - (1986) 132:541-551; Shumaker, S.A., et al., Am Rev Respir Dis (1986) 134:210-213; Wards, B.J., et al., J Clin Microbiol (1987) 25:2309-2313; Whipple, D.L., et al., J Clin Microbiol (1987) 251511-1515).

The results from these prior art methods of genetic analysis have not always been satisfactory. For example, M. paratuberculosis recovered from a Crohn's patient was shown to be over 90% homologous with an M. avium serotype, a higher degree of homology than found between some strains of M. avium. Furthermore, the members of the MAIS complex showed lesser degrees of homology--M. avium and M.

intracellulare showed only 60% DNA homology; M. scrofulaceum showed less than 50% hybridization with either of these strains. Also, although characterization using restriction endonuclease patterns has been applied to mycobacteria, as set forth in the above-cited references, these patterns are uncertain due to deficiencies in the techniques. Extraction of the DNA was generally conducted in aqueous solution, a condition known to shear high molecular weight DNA (e.g., phenol:chloroform:isoamyl alcohol) and conventional gel electrophoresis techniques are limited to resolution of DNA molecules of up to 30-50 kb at the highest MW end of the spectrum. Therefore, only restriction enzymes which generate DNA fragments of roughly 0.5-10 kb can conveniently be used, and many small bands appeared as smears.

The difficulties in this conventional approach have been noted in the literature (McFadden, J.J., et al., J Clin Microbiol (1987) 25:796-801 (supra)), but it has also been asserted that restriction enzyme patterns permit differentiation of mycobacterial species, and that the technique is sensitive enough to detect mycobacterial strain differences (Patel, R., et al., J Gen Microbiol (1986) 132:541-551; Shumaker, S.A., et al., Am Rev Respir Dis (1986) 134:210-213 (both supra)). However, when ribosomal DNA probes were used to detect the restriction fragment patterns, it was not possible to detect differences between M. paratuberculosis isolated from a patient with Crohn's disease from that isolated from stump-tail Macaques, although minor differences were obtained between M. paratuberculosis and M. avium. A distinction has also been found between M. paratuberculosis and M. avium serovars 2 and 3.

A number of pulsed-field gel electrophoretic techniques have been described which permit effective separations of very large fragments of DNA. Pulsed-field gel electrophoresis has been described, for example, by Schwartz, D.C., et al., Cell (1984) 37:67-75, and applied in a number of contexts (Birren, B.W., et al., Nucleic Acids Research (1988) 16:7563-7582; Clark, S.M., et al., Science (1988) 241:1203-1205), demonstrating that fragments as large as 9000 kb can be successfully fractionated using this technique. In addition, pulsed-field electrophoresis has been applied in a single dimension as described by Carle, et al., U.S. patent 4,727,251; Science (1986) 232:65-68. Application of these techniques to genetic analysis of microorganisms is also described generally in WO87/02065.

The present invention provides a more definitive and successful approach to identification and characterization of mycobacteria than is found in the above-described art.

## Disclosure of the Invention

The invention provides improved methods for differentiating among various species and strains of mycobacteria. Application of the method of the invention as illustrated herein provides reference libraries of fragmentation patterns for comparison to results obtained in future conduct of the method. Obtention of the herein reference library and additional data with regard to other strains and species is made possible by a combination of improvements which results in suitably characterizing patterns.

The invention method requires subjecting the chromosomal DNA of a mycobacterial culture to at least one restriction endonuclease followed by separation of the resulting fragments according to size. The size separation is conducted by pulsed-field gel electrophoresis techniques permitting simple digestion patterns to yield relatively large fragments. The DNA is obtained by embedding the mycobacterial cells in agarose plugs and extracting other cellular components, thus leaving the intact DNA behind. The plugs are then treated directly with restriction endonucleases and subjected to pulsed-field gel electrophoresis, and stained with ethidium bromide. The growth conditions for the cultures are also regulated to obtain enhanced DNA isolation effectiveness.

Accordingly, in one aspect, the invention is directed to a method to characterize a strain or species of mycobacteria which method comprises subjecting the chromosomal DNA of the cells in a mycobacteria culture, from which other cellular components have been removed, to digestion with at least one restriction endonuclease to obtain fragments, followed by separation of the resulting fragments according to size, and detecting the fragments. The separation is generally conducted using pulsed-field gel electrophoresis and the fragments are detected using a suitable dye such as ethidium bromide. The DNA for electrophoresis is obtained by embedding the cells in gel plugs compatible with the electrophoresis gel and extracting all other cellular components leaving the DNA in the plug for direct application to the electrophoresis gel.

## Brief Description of the Drawings

Figure 1 shows the restriction fragment pattern obtained from five species of Mycobacterium after SspI digestion, when subjected to pulsed-field gel electrophoresis.

Figure 2 shows the restriction fragment pattern from bovine and human isolates after digestion with SspI and separation by pulsed-field gel electrophoresis.

Figure 3 shows a comparison of SspI restriction fragment patterns of bovine isolates with M. avium.

Figure 4 shows the restriction fragment pattern from M. phlei after SspI digestion, when subjected to pulsed-field gel electrophoresis.

Detailed Description of the Invention

As used herein, "pulsed-field gel electrophoresis" refers to a separation technique suitable for large fragments of DNA by application of nonhomogeneous fields across an electrophoresis gel, under conditions of gel porosity where the fragments to be separated are retarded by the gel. Application of asymmetric patterns can be applied either in a single dimension or using a transverse pattern of field application as described in the Background section above. A variety of protocols and patterns of field application can be employed as is understood in the art.

In the application of the method of the invention to mycobacteria, cultures from biological samples to be tested are grown under culture conditions which provide for ease of extraction of DNA. Typically, the strains are grown as static broth cultures in Middlebrook 7H9 medium, pH 5.9, supplemented with Middlebrook OADC enrichment (GIBCO D3001) and, depending on the strain, 2 mg/l mycobactin-J (Allied Laboratores, CO) is added to the medium. Inocula are obtained to scale up production, and in the final culture, D-cycloserine is added to yield a final concentration of 1 mg/ml when the turbidity at 540 nm reaches 0.2-0.5. (D-cycloserine weakens cell walls by competing with D-alanine in peptidoglycan synthesis; other cell wall-weakening agents could also be used.) The pH is dependent on the strain, and growth is generally conducted at 37°C.

The cells are harvested by centrifugation and suspended in a buffer salt solution, preferably 50 mM Tris HCl, pH 8.0: 100 mM EDTA: 150 mM NaCl (TEN), at about 100-200 mg wet weight/ml and digested with lipase for at least 2 hr at 37°C. An equal volume of 1.5% low gelling temperature agarose is then added, along with 0.25 M EDTA to the cell suspension and the mixtures are gelled in insert molds.

After cooling to 4°C, the molds are removed and placed into 1.0 ml buffer per insert. The buffer contains enzymes for digestion of the cellular components. In a typical preparation, the buffer contains 6 mM Tris HCl, pH 7.6; 1 M NaCl, 0.5% Brij-58; 100 mM EDTA, pH 7.5; 0.2% deoxycholate; 0.5% Sarkosyl; 20 ug/ml DNase-free RNase and 2 mg/ml lysozyme. The inserts are incubated for about 24 hr at 37°C and treated further with digesting media. A typical medium constitutes 20 volumes of 0.5 M EDTA, pH 9.0; 1% Sarkosyl and 2 mg/ml proteinase-K. The molds are then incubated at 50°C for 48 hr with shaking. The digested inserts can then be stored at 4°C in this solution, or can be washed with buffer containing a protease inhibitor, preferably phenylmethyl2sulfonyl fluoride (PMSF), further washed in buffer to remove PMSF, and used for restriction endonuclease digestion and electrophoresis.

Restriction endonuclease digestion is conducted directly on the inserts using standard conditions in buffer containing EDTA, spermidine HCl and a stabilizing protein such as BSA. The inserts are incubated on ice for sufficient time to allow the enzymes to diffuse into the blocks, and then incubated at the temperature recommended for the particular enzyme according to the manufacturer's instructions.

The digested inserts are then subjected to pulsed-field gel electrophoresis using commercially available apparatus to obtain the restriction pattern.

The following examples are intended to illustrate but not to limit the invention.

Example 1

Culture of Mycobacterium Strains

Primary cultures are obtained as primary isolates, frozen cells, or lyophilized cells.

Mycobacteria were isolated from bovine fecal material by a modification of the method described in "Laboratory Methods in Veterinary Mycobacteriology" (1985) USDA, Ames, Iowa. The sample was incubated for 48 hr at room temperature to permit fungal spore germination, and a 1 g aliquot of the sample then added to 40 ml 0.3% (w/v) benzalkonium chloride in a capped centrifuge tube and mixed on a rotator for 30 min. The mixture was incubated for 2 hr at room temperature and approximately 2-3 ml of supernatant above the sediment was removed into a test tube and incubated for an additional 18 hr at room temperature. The resulting supernatant, free of sediment, was used to inoculate agarose slants containing Herrold's egg yolk medium (HEYM) with and without mycobactin-J at 4 drops/slant. Colonies appear after 6-7 weeks incubation at 37°C.

Lyophilized cultures are revived according to the recommended protocol supplied by the American Type Culture Collection which comprises resuspending the dried cells in the appropriate medium and incubation at 37°C without shaking. These cultures are maintained as frozen (-80°C) cell suspension.

The primary cultures were then grown as broth cultures as follows:

M. avium and M. paratuberculosis were grown in Middlebrook 7H9 medium, pH 5.9 supplemented with Middlebrook OADC enrichment (GIBCO D3001) and 2 mg/l mycobactin-J. Single colonies or a 1 ml thawed cell suspension were used to inoculate a final volume of 10 ml medium in a 25 cm² tissue culture flask. Incubation was at 37°C. When a turbid culture was obtained, 1 ml was used to inoculate 50 ml of medium in a 175 cm² tissue culture flask. D-Cycloserine was added to the medium to yield a final concentration of 1 mg/ml at turbidity of 0.2-0.5 at 540 nm. Incubation at 37°C was then continued for another 18-24 hr.

For cultures of M. phlei, mycobactin-J was omitted from the medium which was maintained at pH 6.7, and the cultures were incubated as 100 ml volumes in 1 l Erlenmeyer flasks. A shorter period of incubation after D-cycloserine addition, of 2.5-4 hr, was used.

## Example 2

### Preparation of Inserts

Inserts for pulsed-field gel electrophoresis were prepared by modifications of the methods described by Whipple, D.L., et al., J Clin Microbiol (1987) 25: 1511-1515; Schwartz, D.C., et al., Cell (198 ) 37:67-75, and LKB Instruction Manual for the LKB 2015 Pulsaphor™ system.

Briefly, the cells were harvested by centrifugation at 1500 x g for 10 min at 4°C, resuspended in 50 mM Tris HCl, pH 8.0: 100 mM EDTA: 150 mM NaCl (TEN) and pelleted. The cells were resuspended in TEN at 100-200 mg wet weight/ml and lipase (Sigma L-4385) was added to yield a final concentration of 20,000 units/ml. The suspension was incubated for a minimum of 2 hr at 37°C and an equal volume of 1.5% low-gelling temperature agarose (Sea Plaque) dissolved in 0.25 molar EDTA was added. The mixture was dispensed into insert molds 2 x 5 x 10 mm.

## Example 3

### Removal of Cellular Components

The method was a combination of that described by Smith, C.L., et al., "Genetic Engineering" (1986) 8:45-71, Plenum Press, Newark, NJ, and New England Biolab 1988-1989 catalog.

Briefly, the inserts were cooled to 0°C and removed from the molds using a flame-sealed Pasteur pipette, and placed into 0.5 ml buffer containing 6 mM Tris HCl, pH 7.6; 1 M NaCl; 0.5% Brij-58; 100 mM EDTA, pH 7.5; 0.2% deoxycholate; 0.5% Sarkosyl; 20 ug/ml DNase-free RNase; and 2 mg/ml lysozyme. The inserts were incubated in buffer 24 hr at 37°C, and then placed in a solution containing 20 volumes of 0.5 M EDTA, pH 9.0; 1% sarkosyl; 2 mg/ml proteinase-K. These solutions were incubated at 50°C for 48 hr with gentle shaking. The inserts could be stored at 4°C in this solution.

## Example 4

Restriction Enzyme Analysis of Mycobacterium Strains

A. Inserts containing DNA extracted as described in Examples 1-3 from M. phlei ATCC 11758, ATCC 27086 and ATCC 35784 were digested with the restriction endonuclease SspI as follows. The inserts were washed twice for 1 hr at room temperature in 10 volumes of buffer containing 10 mM Tris HCl, pH 8.0, 1 mM EDTA containing 1 mM phenylmethylsulfonyl fluoride (PMSF) and 3 times in buffer (10 mM Tris HCl, pH 8.0, 100 mM EDTA, pH 8.0) without PMSF. The inserts were washed in 50 volumes TE buffer (10 mM Tris HCl, pH 8.0, 0.1 mM EDTA, pH 8.0) for 30 min. Digestions were carried out in 1.5 ml microcentrifuge tubes using buffer as recommended by the manufacturer, containing spermidine HCl at 4 mM and bovine serum albumin at 100 ug/ml. The inserts were added last to the reaction tubes which were incubated on ice for 30 min to allow the endonucleases to diffuse into the blocks; the reactions were then placed at the recommended temperature for 3 hr.

The inserts were loaded directly onto an LKB Pulsaphor™ apparatus fitted with a hexagonal electrode array. Lambda virus DNA oligomers were used as molecular weight standards. The gels were cast of 1% agarose in Tris-borate-EDTA buffer and electrophoresis was performed at constant voltage of 170 volts with a 10 second pulse time and buffer temperature of 12 °C for 20 or 40 hr.

The gels were stained for 30 min in 1.5 l of water containing 1 mg/l ethidium bromide and photographed under ultraviolet light at 300 nm using Polaroid 665 film.

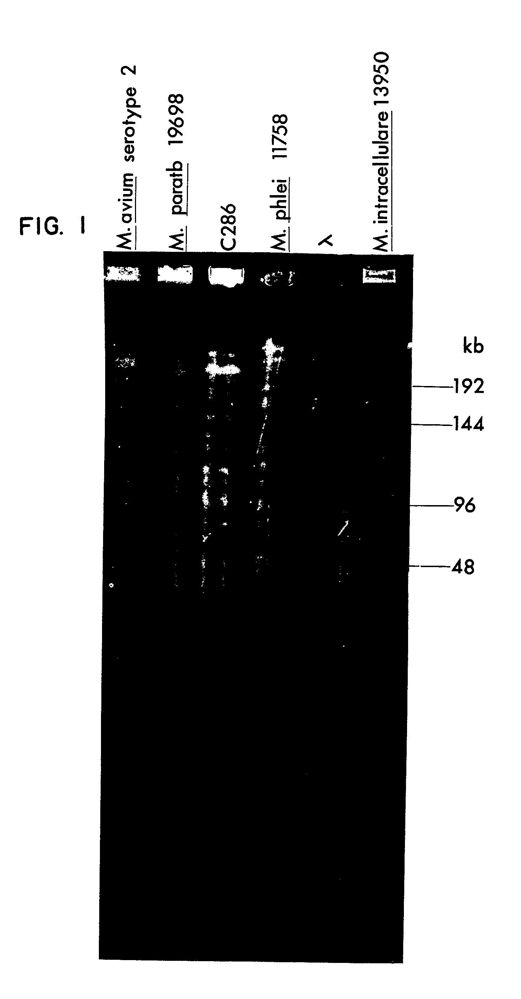

B. Using the procedure of paragraph A, inserts containing SspI digests of M. avium serotype 2, ATCC 35712, M. paratuberculosis, strain ATCC 19698, and strain C286 supplied by Dr. E.A. Sugden, Animal Diseases Research Institute, Nepean, Ontario, M. phlei, strain ATCC 11758 and M. intracellulare, strain ATCC 13950, were prepared and subjected to the pulsed-gel procedure for 20 hr as above-described. The results are shown in Figure 1.

As shown in the figure, five groups of overall banding patterns are clearly visible; one for each species studied. Lane 1: M. avium serotype 2 (ATCC 35712); lane 2: M. paratuberculosis ATCC 19698; lane 3: M. paratuberculosis C286; lane 4: M. phlei ATCC 11758; lane 5: lambda concatemers; lane 6: M. intracellulare ATCC 13950. The restriction fragment patterns can also be described in terms of the lengths in kilobases (kb) of each of the bands in the individual patterns. The patterns are thus described in Table 1.

Table 1

| Fragment Lengths of Restriction Fragment Pattern Bands* | | | | |
|---|---|---|---|---|
| M. avium 2 | M. paratuber culosis | C286 | M. phlei | M. intracellulare |
| | | 192 | | |
| | 182 | | 182 | |
| 170 | | 170 | | |
| | | | 162 | 162 |
| 153 | 152 | | 152 | |
| | | | | 150 |
| | | 142 | | |
| | | 140 | 140 | 140 |
| 135 | | 135 | 135 | |
| | 132 | | | |
| | 127 | | | |
| | | 128 | 128 | |
| 120 | | | | |
| | | | 118 | |
| | | 117 | | |
| | | | | 115 |
| | 114 | | | |
| | | 113 | 113 | 113 |
| | 112 | | | |
| | 110 | | | 110 |
| | | | 90 | |
| | | | | 80 |
| | 68 | | | |
| | 60 | | | |
| | | | 48 | |

* approximate fragment lengths in kilobases (kb)

Bands outside the range of the standard curve are not included. While the emphasis is on comparing total banding patterns, some individual bands can be used as indicators of a particular species. For example, the 132 kb band distinguishes M. paratuberculosis 19698 from M. avium serotype 2 which has a 135 kb band in the same region.

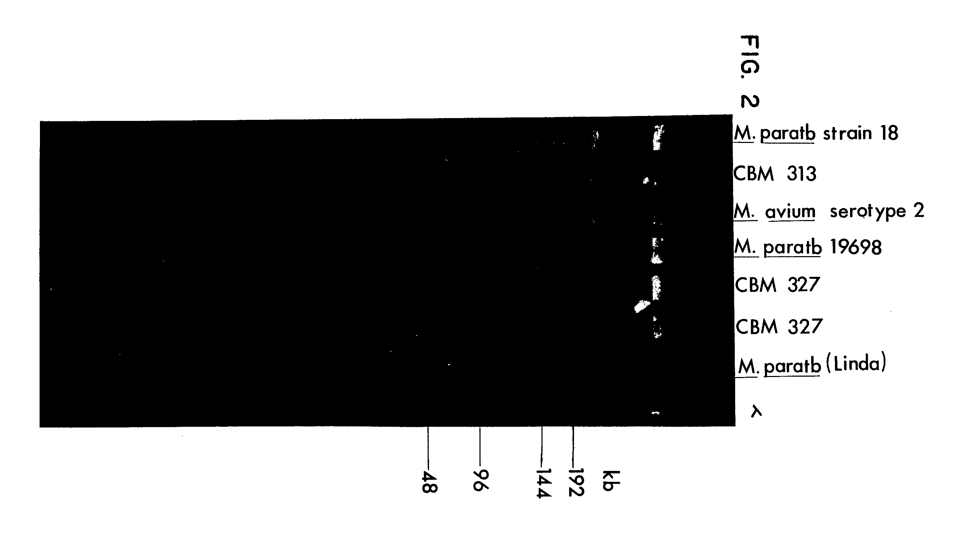

C. Using the procedure of paragraph A, inserts containing SspI digests of M. paratuberculosis strain 18 (supplied by W. D. Richards, Allied Laboratories, CO), CBM 313, M. avium serotype 2, ATCC 35712, M. paratuberculosis strain ATCC 19698, CBM 327 (two separate batches of inserts) and M. paratuberculosis strain Linda, ATCC 43015 were prepared. Strains CBM 313 and CBM 327 are primary isolates from cows diagnosed as having Johne's disease. The results of a 20 hr pulsed-field gel electrophoresis run are shown in Figure 2. Lane 1: M. paratuberculosis strain 18; lane 2: primary bovine isolate CBM 313; lane 3: M. avium serotype 2 (ATCC 35712); lane 4: M. paratuberculosis 19698; lane 5: primary bovine isolate CBM 327; lane 6: primary bovine isolate CBM 327; lane 7: M. paratuberculosis strain Linda; lane 8: lambda concatemers.

As shown in the figure, M. paratuberculosis strain 18 gives a pattern more similar to that of M. avium serotype 2 than to that of M. paratuberculosis ATCC 19698. The pattern of M. paratuberculosis ATCC 19698 is clearly distinct from that of M. avium serotype 2. Therefore, reclassification of M. paratuberculosis strain 18 as M. avium should be considered. The two primary isolates, CBM 313 and CBM 327, show dissimilar patterns--that of CBM 327 resembles ATCC 19698; that of CBM 313 resembles that of M. avium serotype 2. (Prior application of classical techniques indicated CBM 313 to be a strain of M. paratuberculosis.)

The human isolate, M. paratuberculosis strain Linda (ATCC 43015), was originally cultured from a patient having Crohn's disease. As seen in Figure 2, this isolate has a DNA fragment pattern similar to that of both M. paratuberculosis 19698 and the primary bovine isolate CBM 327. This approach, therefore, is useful in speciating human isolates of mycobacteria with potential clinical significance.

7

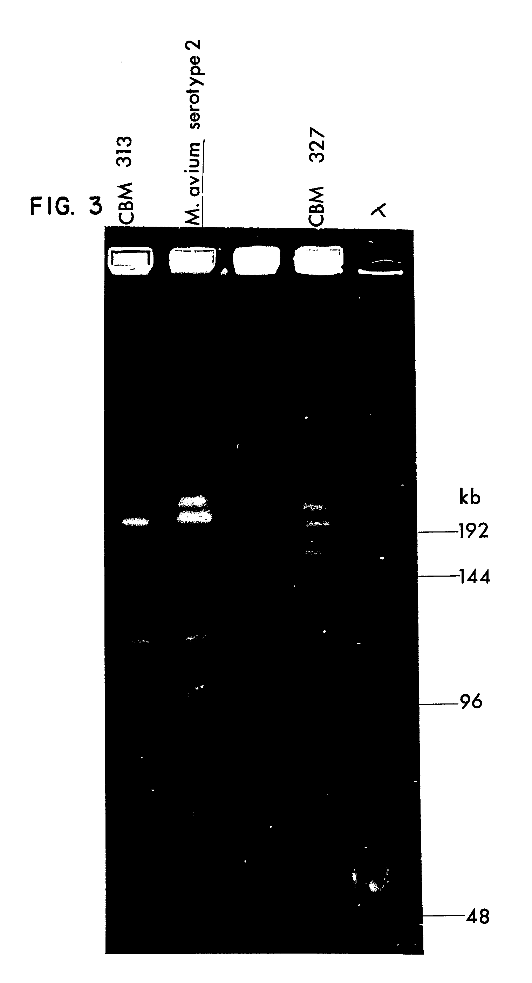

The comparison of CBM 313 and CBM 327 with M. avium serotype 2, after 40 hr of pulsed-field gel electrophoresis, is also seen in Figure 3. Lane 1: primary bovine isolate CBM 313; lane 2: M. avium serotype 2 (ATCC 35712); lane 3: blank; lane 4: primary bovine isolate CBM 327; lane 5: lambda concatemers. This figure illustrates that CBM 313 has minor restriction fragment length differences when compared with M. avium serotype 2. For example, there is an extra band in the 110 kb region of CBM 313 pattern which is not present in the M. avium serotype 2 pattern.

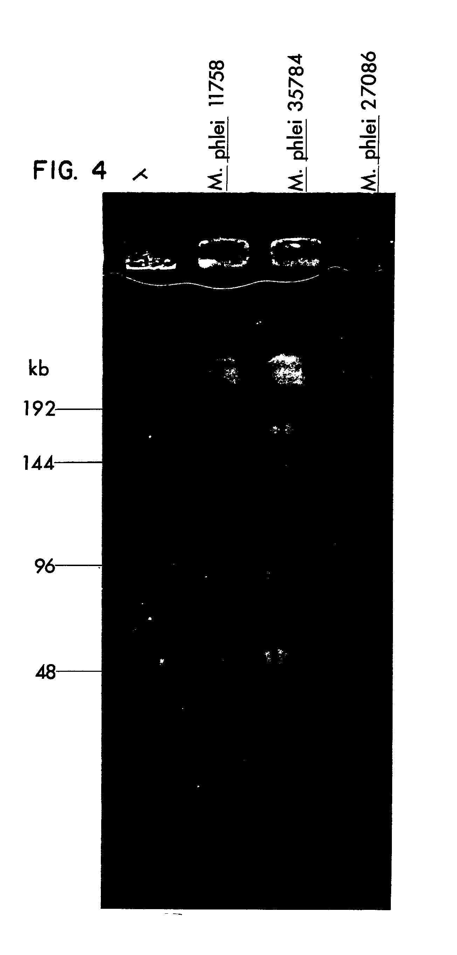

D. Using the procedure of paragraph A, inserts containing SspI digests of three strains of M. phlei - (ATCC 11758, ATCC 35784 and ATCC 27086) were prepared. The results are shown in Figure 4. Lane 1: lambda concatemers; lane 2: M. phlei (ATCC 11758); lane 3: M. phlei (ATCC 35784); lane 4: M. phlei - (ATCC 27086).

As shown in the figure, there is a large degree of similarity between the patterns. However, unique bands were also resolved and are most readily seen in the 100 kilobase region. The distinguishing bands become identification markers for the individual strains of M. phlei. The banding patterns are described in terms of fragment lengths in kilobases in Table 2.

Table 2

| Fragment Lengths of Restriction Fragment Pattern Bands* | | |
|---|---|---|
| M. phlei (ATCC 11758) | M. phlei (ATCC 35784) | M. phlei (ATCC 27086) |
| 127 | 127 | |
| | | 125 |
| 117 | | |
| 107 | 103 | |
| 91 | 91 | |
| | | 86 |
| 52 | 52 | |
| | | 50 |

* approximate fragment lengths in kilobases (kb)

This approach, therefore, can be used to distinguish members of an individual mycobacterial species.

**Claims**

1. A method to characterize a strain of mycobacteria which method comprises subjecting the chromosomal DNA obtained from cells of a mycobacteria culture to digestion with at least one restriction endonuclease; followed by
separating the resulting fragments according to size; and
detecting the resulting fragments.

2. The method of claim 1 wherein said separating is conducted using pulsed-field gel electrophoresis.

3. The method of claim 1 or 2 wherein said detecting comprises treating the separated fragments with a dye.

4. The method of claim 3 wherein the dye is ethidium bromide.

5. The method of any one of the preceding claims wherein the restriction nuclease is SspI.

6. The method of any one of the preceding claims wherein the chromosomal DNA is prepared by
treating a suspension of the cells which is contained in a gelled solid medium with reagents to effect solubilization of non-DNA cellular components; and
washing the gelled solid to remove said reagents and solubilized cellular components so that said chromosomal DNA is retained in the gelled solid.

7. The method of claim 6 wherein said reagents include at least one lipase, at least one protease, at least one detergent, and RNase.

8. The method of claim 6 or 7 wherein the chromosomal DNA retained in the gelled solid is subjected to digestion with at least one restriction endonuclease by contacting said gelled solid with the endonuclease and the separating is conducted by subjecting the resulting gelled plug to pulsed-field gel electrophoresis.

FIG. I

FIG. 2

FIG. 3

FIG. 4

λ

M. phlei 11758

M. phlei 35784

M. phlei 27086

kb

192—

144—

96—

48—